# FASCICULE DE BREVET EUROPEEN

(11) **EP 1 967 187 B1**
(45) Date de publication et mention de la délivrance du brevet: **06.04.2011**
(21) Numéro de dépôt: 07447015.4
(22) Date de dépôt: 06.03.2007
(51) Int. Cl.: A61K 31/198, A61K 31/7048, A61P 29/00

(54) **Composition à base de rutine et de L-lysine**
Zusammensetzung auf Basis von Rutin und L-Lysin
Composition based on rutin and L-lysine

(43) Date de publication de la demande: 10.09.2008
(73) Titulaire: Ennamany, Rachid, 33800 Bordeaux (FR)
(72) Inventeur: Ennamany, Rachid, F-33800 Bordeaux (FR); Mossalayi, M. Djavad, F-33000 Bordeaux (FR)
(74) Mandataire: Powis de Tenbossche, Roland

(56) Documents cités:
- EP-A2- 0 085 579
- EP-A2- 0 420 232
- DE-A1- 4 243 363
- US-A1- 2004 234 517
- DATABASE WPI Week 200430 Derwent Publications Ltd., London, GB; AN 2004-326072 XP002448742 -& RU 2 217 138 C1 (BALAVADZE M E) 27 novembre 2003 (2003-11-27)
- DATABASE WPI Week 200554 Derwent Publications Ltd., London, GB; AN 2005-526710 XP002448743 & JP 2005 198642 A (MIYAUCHI Y) 28 juillet 2005 (2005-07-28)
- GUARDIA TERESITA ET AL: "Anti-inflammatory properties of plant flavonoids. Effects of rutin, quercetin and hesperidin on adjuvant arthritis in rat." FARMACO (LAUSANNE), vol. 56, no. 9, septembre 2001 (2001-09), pages 683-687, XP002445621 ISSN: 0014-827X
- MORIKAWA KEIKO ET AL: "Inhibitory effect of quercetin on carrageenan-induced inflammation in rats." LIFE SCIENCES, vol. 74, no. 6, 26 décembre 2003 (2003-12-26), pages 709-721, XP002445622 ISSN: 0024-3205
- LI X ET AL: "Separation and determination of rutin and quercetin in the flowers of Sophora japonica L. by capillary electrophoresis with electrochemical detection" CHROMATOGRAPHIA 2002 GERMANY, vol. 55, no. 3-4, 2002, pages 243-246, XP002445623 ISSN: 0009-5893

## Description

La présente invention a pour objet l'utilisation de la L-lysine ou un sel ou ester acceptable de la L-lysine pour la préparation d'une composition pour un traitement anti-inflammatoire comprenant de la rutine ou un dérivé glycolyse de la rutine.

On connaît des compositions comprenant de la rutine ou des dérivés de rutine.

La rutine est un composé de formule 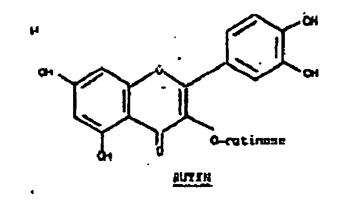 avec la O - rutinose répondant à la formule

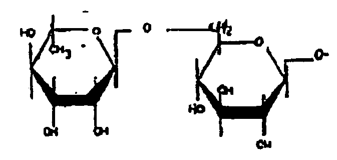

Selon le Merck Index (1983 - 10ème édition), la rutine est connue pour réduire la fragilité capillaire.

Par le document EP-A-420232 (brevet européen déchu), on connaît des dérivés particuliers de bioflavonoïdes de formule 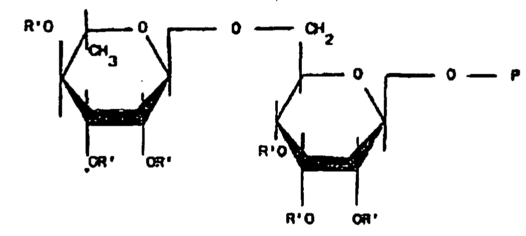 dans laquelle P représente le résidu flavonique contenant des hydroxyles phénoliques et R' représente H, (CO-R-CO-O-X), où X est sélectionné parmi H, le cation d'une base acceptable pharmaceutiquement et les radicaux de polyoxyéthylène glycols, et de polyoxypropylène glycols et leurs O-monométhyléthers et R est un radical alkyle ou aryle d'un acide bicarboxylique, étant donné qu'au moins un substituant R' est différent de H.

Ainsi que stipulé dans ce brevet, la rutine ou (3-rutisonide de 3,3',4',5,7-penthahydroxyflavone) est utilisée pour sa propriété de réduire la fragilité des vaisseaux capillaires.

Selon ce brevet, les composés présentent des propriétés pour réduire l'oedème induit par l'administration dans la surface plantaire d'un rat d'une solution de histamine, pour réduire l'inflammation des yeux de lapins, pour traiter des troubles gynécologiques, pour traiter des troubles cérébrovasculaires.

Selon ce brevet, lesdits dérivés peuvent être utilisés pour la préparation de compositions pharmaceutiques pour administration par voie orale, par exemple via des capsules, des comprimés, des solutions, par voie parentérale et par voie topique.

Par le document WO03/033007 (demande de brevet pour laquelle la phase européenne n'a pas été engagée), on connaît des compositions pharmaceutiques à usage thérapeutique contenant des extraits de plantes médicinales qui présentent des propriétés cicatrisantes et anti-inflammatoires. Selon ce document, la composition doit contenir des extraits de Matricaria recutita (têtes de camomille), Althaea Officinalis L. (racine), Malva sylvestris (fleur), Tillia Platyphyllos (fleurs de tilleuls à grande feuilles) et Achillea millefolium L. (fleurs).

Par le document US-A-2006/0034954, on connaît des compositions médicales, sous forme de compléments alimentaires, qui contiennent des fibres alimentaires et lignan, des carboxyhydrates, des graisses et des modulateurs de graisse, des protéines, des phytoestrogènes, des flavonoïdes, des isoflavones, des agents potentialisant l'activité d'isoflavone, des vitamines, des composés activant la méthylation, des aminoacides (tels qu'un mélange de L-lysine, L-thréonine, triméthylglycine et N-acethylcystéine), des phytonutriments.
La L-lysine est connue par le Merck Index de 1983 (10ème édition) comme étant un additif alimentaire, la L-lysine pouvant se présenter sous la forme de sels ou esters. La lysine répond à la formule C6H14N2O2 :

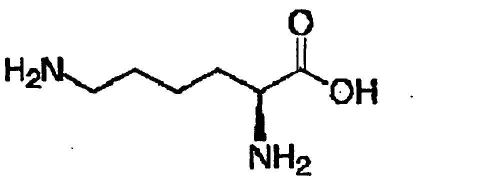

Le document "Anti-inflammatory properties of plant flavonoids. Effects of rutin, quercetin and hesperidin on adjuvant arthritis in rat" Guardia et al, Il Farmaco 56 (2001) décrit l'activité anti-inflammatoires de trois flavonoïdes, à savoir la rutine, la quercetine et l'hesperedine. Après 7 jours de traitement la réduction du volume de l'inflammation est au maximum d'environ 50% pour les rats recevant quotidiennement une dose de 80mg de rutine/kg.

Le document "Inhibitory of quercetin on carrageenan induced inflammation in rats" Morikawa et al, Life sciences 74 (2003) décrit l'effet de la quercetine sur la réponse inflammatoire induite par du carrageenan.

La demande US2004/0234517 a pour objet des compositions pour le traitement de l'asthme, d'allergies ou de maladies inflammatoires. Selon ce document, l'activité de l'arginase peut être inhibée par de nombreux acides aminés, par exemple la lysine, tandis que l'inhibition de ARG1 pourrait améliorer les maladies inflammatoires.

L'inflammation est la réponse des tissus vivants vascularisés aux agressions (blessures, infections, pathologies). Elle n'est en soi « qu'un symptôme ». Même si ses signes cliniques ne sont pas toujours perceptibles extérieurement, une inflammation se manifeste en général par 4 signes principaux :
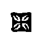 Rougeurs
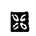 Chaleur
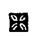 Tuméfaction (gonflement)
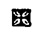 Douleurs

Les douleurs, fièvres et raideurs musculaires peuvent être traitées par la prise d'anti-inflammatoires. Et lorsqu'elle n'est pas guérie, elle peut devenir une maladie inflammatoire chronique comme :
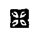 La polyarthrite rhumatoïde (articulations)
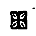 La sclérose en plaque (qui atteint le système nerveux central)
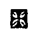 La maladie de Crohn (intestins)

La réaction inflammatoire est complexe et fait intervenir de nombreux phénomènes immunitaires. De nombreuses substances présentes dans le sang en sont le reflet, par exemple :
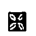 Des hormones (bradykinine, histamine, sérotonine, mélatonine, ...)
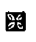 Des prostaglandines (thromboxane, Pg E₂, ...)
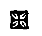 Des cytokines (TNF-α, IL-1, IL-6, etc......
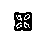 Des radicaux oxygénés tels que le monoxyde d'azote (NO)

Les traitements actuels des maladies inflammatoires ont pour but de :
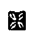 Freiner et si possible d'arrêter l'évolution de la maladie
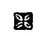 De préserver les fonctions des organes touchés
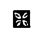 De diminuer les douleurs consécutives à la pathologie

Les traitements comportent les traitements symptomatiques et les traitements de fond.

Les traitements symptomatiques sont destinés à calmer rapidement la douleur et des manifestations cliniques de l'inflammation, mais ne s'attaquent pas aux causes mêmes de l'inflammation. Dans de tels traitements, on utilisera les médicaments suivants :
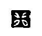 Les médicaments qui permettent de soulager rapidement la douleur, par exemple le paracétamol (Doliprane®, Dafalgan®, Efferalgan®) parfois associés à la codéine pour accroître l'efficacité. Ces antalgiques sont parfois associés à des molécules agissant sur les centres de la douleur du cerveau (Diantalvic®, Propofan®). D'autres encore utilisent des dérivés morphiniques qui sont assez mal tolérés par l'organisme et peuvent engendrer des phénomènes de dépendance.
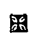 Les anti-inflammatoires stéroïdiens (corticoïdes) à base de cortisone et les anti-inflammatoires non stéroïdiens (A.I.N.S. sans cortisone) tels que l'ibuprofène (Antarène®), le kétoprofène (Ketum®), l'acide niflumique (Nifluril®) ou le diclofénac (Voltarène Emulgel®). Leurs effets néfastes sur l'estomac (ulcères gastriques ou duodénales) empêchent de les associer entre eux et avec ceux de la famille des salicylés (aspirine).

Les traitements de fond participent à l'extinction des symptômes de l'inflammation mais leur action lente a pour objectif principal de freiner, voir de stopper l'évolution de la maladie.
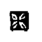 Les médicaments utilisés sont destinés à agir sur des anomalies immunologiques qui créent des lésions de la maladie. Un recul d'au moins trois mois est nécessaire pour juger de leur efficacité. On peut citer les sels d'or (Allochrysine®) dans le traitement de la polyarthrite rhumatoïde, les antipaludéens de synthèse (amodiaquine), les sulfasalazines (Salazopyrine®), les produits thiolés, les immunosuppresseurs dont le méthotrexate (Novatrex®) et enfin les molécules anti-COX (Celebrex®).
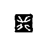 Les médicaments utilisés peuvent également cibler directement un ou des médiateurs (par exemple cytokines) de l'effet inflammatoire. On peut citer par exemple les produits suivants :
   o Anticorps monoclonaux anti-TNF-α dont le chef de file de ces médicaments est le Rémicade® (infliximab), un anticorps anti-TNF-α qui, en association avec des immunosuppresseurs (principalement le méthotrexate), est actuellement très utilisé.
   o Récepteurs solubles du TNF-α tels que l'Enbrel® (étanercept).
   o Récepteurs antagonistes à l'IL-1 tels que le Kineret® (anakinra).

Les problèmes posés par les médicaments actuels pour le traitement de l'inflammation sont divers, par exemple traitement lent, non efficacité, résistance au traitement pour des classes de patients, mais surtout risques élevés d'effets secondaires graves.

Parmi les risques d'effets secondaires graves, on peut citer :
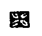 Troubles des systèmes sanguin et lymphatique, c'est à dire des anémies hémorragiques (thrombocytopénie, leucopénie, ...) et des ulcères (aspirine, méthotrexate, sels d'or, ...).
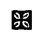 Décalcification et dérèglement des glandes surrénales (corticoïdes).
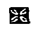 Accidents cardio-vasculaires avec les anti-COX (Vioxx® commercialisé par Merck et retiré du marché dernièrement, ou Celebrex® de Pfizer en pharmacovigilance accrue) qui augmenteraient la synthèse des prostacyclines, anti-agrégeant plaquettaires.
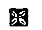 Risques d'infection de type tuberculose extrapulmonaire (étanercept).
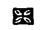 Troubles digestifs (sulfasalazine)
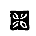 Dépendance.

Un but de l'invention est d'utiliser la L-lysine ou un sel ou ester acceptable de L-lysine pour la préparation d'une composition anti-inflammatoire comprenant de la rutine ou un dérivé glycolyse de rutine, pour laquelle l'activité anti-inflammatoire de la rutine ou dérivé glycolysé de rutine est potentialisée.

Après des années de traitements chimiques non-spécifiques et d'échecs thérapeutiques, les sociétés pharmaceutiques ont développé des approches visant à bloquer in vivo des voie précises (ex : FK506) ou des molécules effectrices de la réponse inflammatoire à l'aide de contre-structures, en particulier d'anticorps monoclonaux. Cette nouvelle classe de médicaments, regroupés sous le nom de bio-thérapeutiques (ex : anti-TNF, anti-IL-1), a fait son apparition pour le traitement des patients atteints de la polyarthrite rhumatoïde ou de la maladie de Crohn, chez qui les traitements traditionnels ont échoué (3). Le principe de ces médicaments est de bloquer la liaison récepteur-ligand par des anticorps spécifiques (ex : l'anti-TNF empêche le TNF-α de se fixer à son récepteur et donc de médier ses effets sur la cellule cible), empêchant ainsi la réaction inflammatoire. Une approche similaire est utilisée pour éliminer les lymphocytes B leucémiques ou inflammatoires par la fixation des anticorps anti-molécules de surface, aboutissant à l'apoptose (mort cellulaire programmée) de la cellule. Cette approche permet une haute spécificité des immunoglobulines mais nécessite des procédés lourds de fabrication (coût annuel de 10 à 15 K€ par patient traité pour la prise en charge de la polyarthrite rhumatoïde par l'anticorps anti-TNF).
De plus, une réponse de l'organisme contre ces molécules peut être associée à ce type de traitement. En effet, la taille relativement importante de ces molécules les rend immunogènes ce qui se traduit par des réactions secondaires au traitement chez les patients surtout pour les pathologies chroniques telles que le MAI.
Enfin, après le retrait très médiatique du Vioxx®, le milieu scientifique médical craint l'apparition potentielle d'effets secondaires chez les patients traités par l'Enbrel®, notamment d'infections type tuberculose extrapulmonaire (le TNF-α étant une molécule anti-infectieuse de premier ordre dans l'organisme humain).

Pendant la dernière décennie, un intérêt croissant a été observé pour les effets bénéfiques de molécules d'origine alimentaire qui ont été décrites pour avoir des actions sur le système immunitaire : les flavonoïdes.

Les flavonoïdes sont des composés polyphénoliques présents dans la plupart des plantes (4). Elles existent dans de nombreuses sources alimentaires et à différentes concentrations. Elles sont à 10 mg/kg dans les carottes, les champignons et les épinards ; à 50 mg/kg dans les oignions, la laitue, le raisin, le thé et les poivrons. L'apport alimentaire journalier est estimé à 23 mg/jour dont 48% est apporté par les oignons et les pommes.

Formule générique des Flavonoïdes

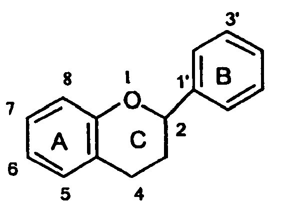

De faible poids moléculaire, elles possèdent trois noyaux phénoliques qui permettent de les classer en familles les flavonols, les flavanols, les flavanones, les flavones, les anthocyanidines et les isoflavones. Les flavonoïdes montrent des effets biologiques variables, selon leurs formules exactes (effets anti-cancéreux, anti-inflammatoires, anti-oxydant, anti-hépatotoxique, cardioprotecteur, apoptotique).

Parmi ces molécules, la quercétine est le flavonol le plus étudié et présente en grande quantité dans les oignons et les pommes et absorbée de façon journalière par l'homme. En plus d'être un inhibiteur de la COX-2, la quercétine diminue la production de TNFα dans de nombreux modèles inflammatoires murin et humain.

Toutefois, la plupart de ces molécules n'ont pas réussi à dépasser le stade expérimental pour manque de conformité aux règles de la pharmacotoxicologie moderne. Ainsi, la quercétine, bien qu'autorisé comme additif alimentaire aux Etats-Unis et dans des pays européens, n'a pas obtenue d'autorisation de la FDA, ni de l'EMEA suite à des résultats controversés de tests de mutagénécité in vitro.

On a remarqué maintenant qu'en associant la rutine (un flavonoïde particulier) ou un dérivé de rutine avec de la L-lysine ou sel ou ester de celle-ci, il était possible de potentialiser des propriétés thérapeutiques ou soulageantes de la Rutine ou dérivé de rutine, en particulier pour le traitement de troubles liés à l'inflammation.

L'invention a pour objet l'utilisation de la L-lysine ou un sel ou ester acceptable de la L-lysine pour la préparation d'une composition pour un traitement anti-inflammatoire comprenant au moins de la rutine ou un dérivé glycolysé de rutine, pour potentialiser l'activité anti-inflammatoire de la rutine ou dérivé glycolysé de rutine. Le dérivé glycolysé de rutine peut être issu par simple extraction de certaines plantes ou peut être synthétisé par un procédé similaire à celui décrit dans EP420232.

De préférence, la composition comprend au moins une quantité efficace de rutine ou dérivé glycolysé de rutine et de L-lysine ou un sel ou ester acceptable de la L-lysine, en tant que principe actif ou mélange de principes actifs, en particulier thérapeutiquement actif(s).

Avantageusement, la composition comprend en outre un ou des excipients acceptables, en particulier pharmaceutiquement acceptables. De tels excipients sont par exemple des excipients pour préparer une composition pour usage topique ou pour administration topique, pour usage ou administration orale, pour usage ou administration parentérale, pour usage ou administration cutanée ou sous cutanée. De tels excipients sont par exemple ....

Selon des formes de réalisation avantageuses, le rapport en poids rutine ou dérivés glycolysés de rutine / L-lysine ou un sel ou ester acceptable de la L-lysine exprimée sous sa forme acide, étant est compris entre 1:1000 et 100:1, avantageusement entre 1:500 et 50:1, de préférence entre 1:250 et 1:1.

Selon un détail avantageux, la composition comprend au moins un dérivé glycolysé de rutine. En tant que dérivé glycolisé de rutine, on peut citer les composés de formule 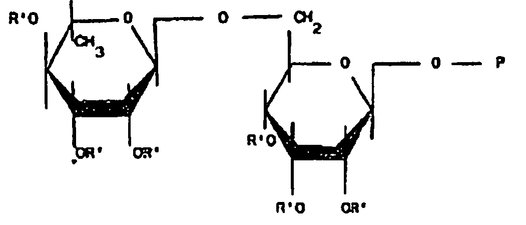 dans laquelle P représente le résidu flavonique de la rutine, résidu de formule : 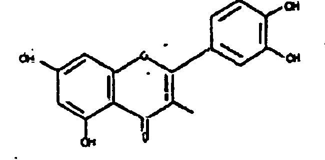 et R' représente H, (CO-R-CO-O-X), où X est sélectionné parmi H, le cation d'une base acceptable pharmaceutiquement et les radicaux de polyoxyéthylène glycols, et de polyoxypropylène glycols et leurs O-monométhyléthers et R est un radical alkyle ou aryle d'un acide bicarboxylique. De façon avantageuse, le dérivé comprend au moins un X représentant un radical de polyoxyéthylène glycol ou un radical de polyoxypropylène glycol ou un monométhyléther de ceux-ci.

De façon avantageuse, la rutine ou le dérivé glycolysé de rutine se présente sous une forme dissoute dans un excipient, avantageusement semi solide ou pâteux. L'excipient ou mélange d'excipient contient avantageusement un polyéthylène glycol ou un dérivé de polyéthylène glycol.

La composition peut se présenter sous une forme injectable, une forme à usage topique, une forme à usage oral ou une forme à usage parentéral. La composition peut se présenter sous une forme de suspensions, de solutions, de poudres, de forme semi-solides, etc.

Selon une forme de réalisation possible, la composition comprend en outre au moins un autre acide aminé que la L-lysine, cet autre acide aminé se présentant sous sa forme acide ou sous la forme d'un sel ou ester. De préférence, dans ce cas, la L-lysine représente la majeure partie du mélange d'acides aminés.

Selon un détail d'une forme de réalisation, la composition comprend au moins un agent antioxydant (tel que la vitamine E) et/ou au moins un inhibiteur de cristallisation (tel que du PVP, HPMC, etc.).

La composition se présente avantageusement sous la forme d'une forme à libération contrôlée. Une telle forme peut par exemple un patch ou support destiné à être appliqué sur la peau d'un patient, ledit patch ou support comprenant une matrice avantageusement adhésive comprenant la rutine ou dérivé glycolysé de rutine et la L-lysine ou sel ou ester de celle-ci. Une telle forme peut également être un comprimé ou une gélule comprenant par exemple une matrice contrôlant la libération de la rutine ou dérivé glycolysé de rutine et/ou de la lysine ou sel ou ester de celle-ci, ou un corps contenant la rutine ou dérivé glycolysé de rutine et/ou de la lysine ou sel ou ester de celle-ci, ce corps étant muni d'au moins une couche de contrôle de libération, avec ou non couche intermédiaire. Une couche pour retarder la libération est par exemple une couche à base de polyacrylate.

Selon un autre détail d'une forme de réalisation, la composition se présente sous la forme d'une matrice dans laquelle la rutine, le ou les dérivés glycolysé de rutine et la 1-lysine ou un sel ou ester acceptable de la L-lysine sont dispersés sous une forme solide, semi solide ou liquide.

Selon encore une autre forme de réalisation possible, la composition comporte un ou des supports inertes ou neutres, en particulier hydrosolubles, qui sont recouvert de particules de rutine ou dérivé glycolysé de rutine et/ou de la L-lysine ou sel ou ester de celle-ci, ces particules étant attachées au moyen d'un polymère hydrosoluble, en particulier la PVP.
Selon une forme de réalisation préférée, la composition comprend au moins un dérivé glycolysé de rutine et

Selon un autre détail d'une composition , elle comprend une quantité efficace de rutine ou dérivé glycolysé de rutine et de L-lysine ou un sel ou ester acceptable de la L-lysine pour soulager ou traiter un ou des troubles liés à l'inflammation et/ou des affections auto-immunes et/ou cutanées, et/ou une quantité efficace de rutine ou dérivé de rutine et de L-lysine ou un sel ou ester acceptable de la L-lysine pour soulager ou traiter des troubles liés au NO ou à une déficience de NO et/ou pour inhiber l'activation et de la prolifération des lymphocytes B (immunité humorale Th-2) et/ou inhiber l'activation et de la prolifération des lymphocytes T (immunité cellulaire Th-1) et/ou inhiber l'activation des macrophages (Liée en majorité à la réponse Th-1) ou la neutralisation de leurs produits.

Selon une particularité avantageuse, la composition comprend au moins un composé choisi parmi les polyoxyéthylène glycols, les polyoxypropylène glycols, les polyoxy(ethylène/propylène) glycols, les éthers de ceux-ci, et leurs mélanges.

Selon un détail d'une forme de réalisation préférée, la composition comprend un dérivé sous forme glycolysée de rutine extrait de Sophorajaponica ou d'une culture de cellules de Sophorajaponica.

Selon toujours un autre détail d'une forme de réalisation préférée, la composition comprend un premier dérivé glycolysé de rutine comportant un ou des radicaux polyoxyglycolisés ou un ester de celui-ci et un deuxième dérivé glycolysé de rutine comportant un ou des radicaux polyoxypropylène glycols ou un ester de celui-ci.

Selon des détails de formes de réalisation, la composition se présente sous une forme où la rutine ou dérivé glycolysé de rutine est séparée de la L-lysine ou sel ou ester de celle-ci par au moins une barrière (pour éviter un contact direct entre la L-lysine et la rutine dans la composition) et/ou sous une forme à libération contrôlée, en particulier une forme pour laquelle la L-Lysine ou sel ou ester de celle-ci est libéré plus rapidement que la rutine ou dérivé glycolysé de rutine.

Des particularités de l'invention ressortiront de la description détaillée suivante, dans laquelle les exemples de réalisation ne sont donnés qu'à titre d'exemples uniquement.

On a extrait des boutons floraux de la Sophora japonica un extrait glycolisé de la 3-rutinoside de 3,3',4',5,7 pentahydroxyflavone. La méthode d'extraction est brièvement décrite ci dessous :
- Porter à ébullition de l'eau distillée dans un ballon surmonté d'un réfrigérant à reflux.
- ajouter à l'eau bouillante par petites fractions, de boutons floraux de S. japonica broyés au mortier.
- porter à ébullition pendant 45 minutes
- filtrer à chaud sur un entonnoir, muni d'un tampon de coton, dans un erlène.
- placer le filtrat dans un réfrigérateur pour obtenir des cristaux.

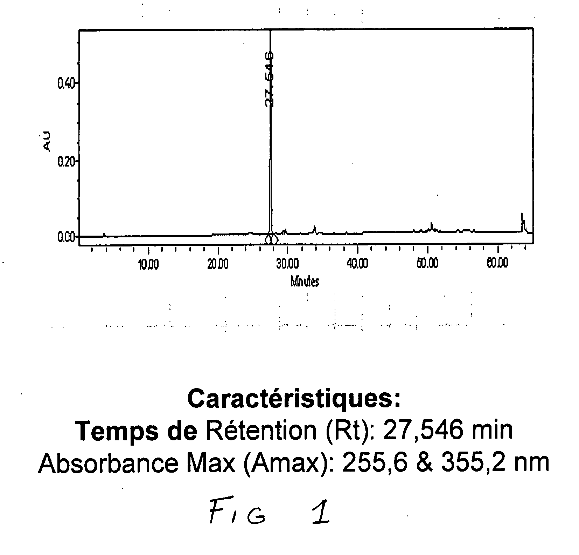

La figure 1 unique est une courbe HPLC de cet extrait. Le temps de rétention du composé dans la colonne est de 27,546 minutes. L'absorbance est maximale pour les longueurs d'onde de 255,6 nm et 355,2 nm.

Des tests ont montré que ce composé possède une action inhibitrice sur la sécrétion des médiateurs pro-inflammatoires par les cellules humaines en réponse aux cytokines Th1.

La L-Lysine présente une action inhibitrice sur l'arginase Th2-dépendante et facilite une réparation tissulaire.

En combinant ces deux composés dans une composition topique de l'invention, on a démontré que cette composition était à l'efficace dans le domaine de l'inflammation, mais également dans le traitement d'affections auto-immunes et/ou cutanées en particulier.

Des tests in vitro ont permis de démontrer la potentialité anti-inflammatoire de cette combinaison et son absence de génotoxicité. L'effet anti-inflammatoire de la combinaison ressort de tests in vitro sur des macrophages murins et humains démontrant une diminution de marqueurs d'inflammation Th1 (inhibition de la production de NO et induction de l'IL-10), tout en inhibant la formation d'arginase. L'arginase, une enzyme pro-inflammatoire peut être induite par la réponse Th2 qui peut être engendrée par la diminution de la réponse Th1. L'inhibition de cette arginase permet d'inhiber la synthèse des molécules protectrices d'agents infectieux, les trypanothiones, et permet ainsi d'éviter certaines infections.

Les tests in vitro sur les macrophages n'ont pas démontré la moindre toxicité de la combinaison sur les macrophages.

Les tests in vitro ont démontré qu'en utilisant la combinaison dérivé glycoside de rutine + L-lysine, il était possible de réduire d'environ au moins 50% la teneur en TNF-α, ainsi que celle de IL-1β et d'au moins environ 30% de MCP-1 dans les surnageants des macrophages murines (après activation) par rapport aux teneurs obtenus pour le dérivé glycoside du milieu seul. En présence de dose de 50 à 500µM de la combinaison, on a également démontré une réduction de plus de 60% de la concentration en nitrite par rapport à l'utilisation d'une composition ne contenant que le milieu de culture, ce qui démontre l'inhibition de la production de NO.

L'utilisation du dérivé glycoside de rutine in vivo dans des rats arthritiques montre une diminution de la teneur en IL-1β dans les sérums de rats traités par rapport à la teneur en IL-1β pour le sang d'un rat témoin (pas d'adjonction de Rutine), démontrant ainsi l'inhibition de la réponse inflammatoire Th1 dans un modèle in vivo approprié de cette réponse. Les résultats in vitro et in vivo démontrent qu'il est possible non seulement de réduire la réponse inflammatoire Th1 par rapport au sang d'un rat témoin. De plus, la composition selon l'invention permet d'éviter ou réduire la réponse inflammatoire, tout en inhibant la formation d'une enzyme pro-inflammatoire, à savoir l'arginase responsable de favoriser certaines infections et donc de régénérer un stimuli inflammatoire, en particulier une réponse Th1.

La combinaison Rutine ou dérivé glucoside de rutine + L-lysine permet ainsi d'inhiber l'activation des macrophages et des kératinocytes liée au cours de la réponse inflammatoire Th1 et Th2 de manière spécifique.

Des compositions préparées selon l'utilisation selon l'invention seront décrites ci-après :

### Exemple 1 : Gélules

Des gélules ont été remplies de compositions contenant un dérivé glycoside de rutine et de la L-lysine avec un rapport en poids L-lysine/dérivé glycolysé de rutine de 50.

Pour cette préparation, le dérivé glycoside de rutine a été mélangé à du PEG fondu.
Une fois le mélange bien homogène, la L-lysine est ajoutée. Des grains de lactose sont ensuite ajoutés au mélange fondu.

Le mélange fondu est ensuite versé dans des gélules pour former des préparations contenant 250mg, 500mg, 750mg de L-lysine et 5mg, 10mg et 150mg de dérivé glycoside de rutine.

De manière similaire, on a préparé des gélules contenant un dérivé glycoside de rutine et de la L-lysine avec un rapport en poids L-lysine dérivé de rutine de 25, 75, 100 et 200. La quantité de L-lysine dans les formulations était respectivement de 100mg, 200mg, 400mg, 600mg et 1000mg.

### Exemple 2 : Comprimés

De la L-lysine, le dérivé glycolysé de rutine, du PVP sont mélangés ensemble en présence d'eau. Du laurylsulfate de sodium est ajouté pour obtenir un mélange ou dispersion plus homgène.

Ce mélange est pulvérisé sur des grains de lactose de 100 à 200µm et séché.

Après séchage, les grains sont comprimés ensemble pour préparer des comprimés contenant respectivement 100mg, 200mg, 500mg et 1000mg de L-lysine. La quantité de dérivé de rutine a été adaptée pour obtenir des comprimés présentant un rapport en poids L-lysine dérivé de rutine de 25, 75, 100 et 200.

Les comprimés sont avantageusement munis d'une pellicule extérieure, par exemple à base de polyacrylate.

Selon une variante possible on prépare deux types de comprimés différents, à savoir un comprimé de dérivé glycolysé de rutine et un autre contenant de la L-lysine. Les comprimés sont alors placés dans un blister.

### Exemple 3 Dispersion de L-lysine et dérivé glycolysé de rutine dans une base cosmétique

la L-lysine et le dérivé glycolysé de rutine sont dispersées dans la base suivante :
- deionized water 75, 61 %
- L-lysine 10,00 %
- mineral oil 9, 00 %
- cetyl alcohol 3, 00 %
- ceteareth - 20 0, 75 %
- dérivé glycolysé de rutine 0, 20 %
- fragrance 0, 15 %
- carbomer 0, 10 %
- methylchloroisothiazoline
   et methylisothiazoline [kathon CG] 0, 065 %
- sodium hydroxide (45 %) 0, 06 %
- butylated hydroxyanisole 0, 06 %
TOTAL 100, 00 %

La composition obtenue montre une dispersion homogène dans la crème et une granulométrie très fine. L'étude de propreté a montré l'absence de germes et champignons ainsi qu'une remarquable stabilité de la composition. Le résultat obtenu ayant été testé dans une étude transcutanée a permis de voir le passage des principes actifs notamment le dérivé glycolysé de rutine à travers le tissu cutané.

### Exemple 4 Crèmes

- phase aqueuse A : eau déminéralisée associée à un produit hydratant
- phase huileuse B : émulsionnant + émolliant + huile
- phase C : conservateur, parfum
- phase D : produit actif: dérivé glycolysé de rutine et L-lysine, sous forme d'une suspension visqueuse ou d'un gel ou d'une poudre sensiblement sèche.

### Exemple 5 Lotions

contenant seulement une phase aqueuse A : eau déminéralisée, propylène glycol, conservateur, parfum et produit actif : sous forme d'une suspension visqueuse ou d'un gel ou d'une poudre sensiblement sèche.

### Exemple 6 Shampooings

contenant seulement une phase aqueuse A à base d'eau déminéralisée, détergents, moussants, épaississants, parfum et produit actif : sous forme d'une suspension visqueuse ou d'un gel ou d'une poudre sensiblement sèche.

### Exemple 7 Gels

- On considère les hydrogels et les oléogels, obtenus par adjonction à la phase aqueuse A ou à la phase huileuse B d'agents de type émulsifiant et épaississant.
- phase C : parfum, conservateur
- phase D : produit actif : dérivé glycolysé de rutine et L-lysine, sous forme d'une suspension visqueuse ou d'un gel ou d'une poudre sensiblement sèche.

### Exemple 8 Solutions

Solutions contenant seulement une phase aqueuse A essentiellement à base d'eau déminéralisée, parfum, conservateur et produit actif : produit actif : dérivé glycolysé de rutine et L-lysine, sous forme d'une suspension visqueuse ou d'un gel ou d'une poudre sensiblement sèche.

### Exemple 9 Laits

- phase aqueuse A : essentiellement à base d'eau désionisée
- phase huileuse B : huile + émulsionnant + émolliant
- phase C : conservateur + produit hydratant
- phase D : produit actif : dérivé glycolysé de rutine et L-lysine, sous forme d'une suspension visqueuse ou d'un gel ou d'une poudre sensiblement sèche.

Dans les exemples donnés ci-dessus, concernant les crèmes, gels ou laits, les différentes phases A, B, C et D, dans des proportions qui peuvent varier entre elles en fonction des applications souhaitées, sont mélangées de manière habituelle, comme le réalise habituellement l'Homme de l'art dans ce domaine.

Concernant les lotions, solutions et shampooings, la composition à usage topique contient les différents constituants, dont on peut varier les teneurs en fonction des applications, mélangés dans la seule phase aqueuse, comme le réalise habituellement l'Homme de l'art dans ce domaine.

### Exemple 10 Patch pour administration transdermale

Une huile silicone a été mélangé avec le dérivé glycolysé de rutine et de la L-lysine.

Ce mélange a été mélangé avec une résine silicone durcissable apte à être adhésive.

Le mélange ainsi obtenu a été versé sur un support faiblement poreux. Après le durcissement de la résine adhésive, un film de protection apte à être retiré facilement est placé sur la résine adhésive.

## Revendications

1. Utilisation de la L-lysine ou un sel ou ester acceptable de la L-lysine pour la préparation d'une composition pour un traitement anti-inflammatoire comprenant au moins de la rutine ou un dérivé glycolysé de rutine, pour potentialiser l'activité anti inflammatoire de la rutine ou du dérivé glycolysé de la rutine.

2. Utilisation selon la revendication 1, **caractérisée en ce qu'**on utilise, pour la préparation de la composition, au moins une quantité efficace de rutine ou dérivé glycolysé de rutine et de L-lysine ou un sel ou ester acceptable de la L-lysine, en tant que principe actif pour le traitement de troubles liées à l'inflammation.

3. Utilisation suivant la revendication 1 ou 2, **caractérisée en ce que** pour la composition, le rapport en poids rutine ou dérivés glycolysé de rutine / L-lysine ou un sel ou ester acceptable de la L-lysine exprimée sous sa forme acide, est compris entre 1:1000 et 100:1, avantageusement entre 1:500 et 50:1, de préférence entre 1:250 et 1:1.

4. Utilisation selon l'une quelconque des revendications précédentes, **caractérisée en ce qu'**on utilise une quantité efficace d'un dérivé glycolysé de rutine.

5. Utilisation suivant l'une quelconque des revendications précédentes, **caractérisée en ce que** la rutine ou le dérivé glycolysé de rutine se présente sous une forme dissoute dans un excipient, avantageusement semi solide ou pâteux.

6. Utilisation suivant l'une quelconque des revendications précédentes, **caractérisée en ce que** la composition se présente sous une forme injectable, une forme à usage topique, une forme à usage oral ou une forme à usage parentéral.

7. Utilisation suivant l'une quelconque des revendications précédentes, **caractérisée en ce que** la composition comprend au moins un autre acide aminé que la L-lysine, cet autre acide aminé se présentant sous sa forme acide ou sous la forme d'un sel ou ester.

8. Utilisation suivant l'une quelconque des revendications précédentes, **caractérisée en ce que** la composition comprend au moins un agent antioxydant.

9. Utilisation suivant l'une quelconque des revendications précédentes, **caractérisée en ce que** la composition comprend au moins un inhibiteur de cristallisation.

10. Utilisation suivant l'une quelconque des revendications précédentes, **caractérisée en ce que** la composition se présente sous la forme d'une forme à libération contrôlée.

11. Utilisation suivant l'une quelconque des revendications précédentes, **caractérisée en ce que** la composition se présente sous la forme d'une matrice dans laquelle la rutine, le ou les dérivés glycolysés de rutine et la 1-lysine ou un sel ou ester acceptable de la L-lysine sont dispersés sous une forme solide, semi solide ou liquide.

12. Utilisation suivant l'une quelconque des revendications précédentes, **caractérisée en ce que** la composition comprend au moins un composé choisi parmi les polyoxyéthylène glycols, les polyoxypropylène glycols, les polyoxy(ethylène/propylène) glycols, les éthers de ceux-ci, et leurs mélanges.

13. Utilisation suivant l'une quelconque des revendications précédentes, **caractérisée en ce que** la composition comprend en tant que dérivé glycolysé de rutine, un dérivé sous forme glycolysée de rutine extrait de Sophora japonica ou d'une culture de cellules de Sophora japonica.

14. Utilisation suivant l'une quelconque des revendications précédentes, **caractérisée en ce que** la composition comprend un extrait de Sophora japonica et/ou un extrait glycolisé de la 3-rutinoside de 3,3',4',5,7 pentahydroxyflavone présentant une courbe HPLC présentant un temps de rétention de 27,546 minutes avec une absorbance maximale pour des longueurs d'onde de 255,6nm et de 355,2 nm.

15. Utilisation suivant l'une quelconque des revendications précédentes, **caractérisée en ce qu'**on utilise une quantité efficace de rutine ou dérivé glycolysé de rutine et de L-lysine ou un sel ou ester acceptable de la L-lysine pour soulager ou traiter un ou des troubles liés à l'inflammation et/ou des affections auto-immunes et/ou cutanées.

16. Utilisation suivant l'une quelconque des revendications précédentes, **caractérisée en ce qu'**on utilise une quantité efficace de rutine ou dérivé glycolysé de rutine et de L-lysine ou un sel ou ester acceptable de la L-lysine pour soulager ou traiter des troubles liés au NO, IL-1, TNF- ou MCP-1.

17. Utilisation suivant la revendication 1, **caractérisé en ce qu'**on utilise un kit pharmaceutique, en particulier sous forme de Blister, comprenant un premier support ou dispositif de réception ou comprimé ou gélule contenant de la rutine ou un dérivé glycolysé de rutine, et un deuxième support ou dispositif de réception ou comprimé ou gélule contenant la L-lysine ou un sel ou ester de celle-ci.

## Claims

1. Use of L-lysine or an acceptable salt or ester of L-lysine for the preparation of a composition for an anti-inflammatory treatment comprising at least rutin or a glycolized rutin derivative, in order to potentiate the anti-inflammatory activity of the rutin or the glycolized rutin derivative.

2. Use according to claim 1, **characterized in that**, for the preparation of the composition, at least an effective quantity of rutin or glycolized rutin derivative and L-lysine or an acceptable salt or ester of L-lysine is used as active ingredient for the treatment of disorders linked to inflammation.

3. Use according to claim 1 or 2, **characterized in that**, for the composition, the ratio by weight of rutin or glycolized rutin derivatives / L-lysine or an acceptable salt or ester of L-lysine expressed in its acid form is comprised between 1:1000 and 100:1, advantageously between 1:500 and 50:1, preferably between 1:250 and 1:1.

4. Use according to any one of the previous claims, **characterized in that** an effective quantity of a glycolized rutin derivative is used.

5. Use according to any one of the previous claims, **characterized in that** the rutin or the glycolized rutin derivative is in a form dissolved in an excipient, advantageously semi-solid or pasty.

6. Use according to any one of the previous claims, **characterized in that** the composition is in an injectable form, a form for topical use, a form for oral use or a form for parenteral use.

7. Use according to any one of the previous claims, **characterized in that** the composition comprises at least one amino acid other than L-lysine, this other amino acid being in its acid form or in the form of a salt or ester.

8. Use according to any one of the previous claims, **characterized in that** the composition comprises at least one antioxidant agent.

9. Use according to any one of the previous claims, **characterized in that** the composition comprises at least one crystallization inhibitor.

10. Use according to any one of the previous claims, **characterized in that** the composition is in a form for controlled release.

11. Use according to any one of the previous claims, **characterized in that** the composition is in the form of a matrix in which the rutin, the glycolized rutin derivative(s) and the L-lysine or an acceptable salt or ester of L-lysine are dispersed in a solid, semi-solid or liquid form.

12. Use according to any one of the previous claims, **characterized in that** the composition comprises at least one compound chosen from the polyoxyethylene glycols, polyoxypropylene glycols, polyoxy(ethylene/propylene) glycols, ethers thereof, and their mixtures.

13. Use according to any one of the previous claims, **characterized in that** the composition comprises, as glycolized rutin derivative, a derivative in the glycolized form of rutin extracted from Sophora japonica or a culture of cells of Sophora japonica.

14. Use according to any one of the previous claims, **characterized in that** the composition comprises an extract of Sophora japonica and/or a glycolized extract of 3,3',4',5,7-pentahydroxyflavone-3-rutinoside having an HPLC curve showing a retention time of 27.546 minutes with a maximum absorbance for wavelengths of 255.6 nm and 355.2 nm.

15. Use according to any one of the previous claims, **characterized in that** an effective quantity of rutin or glycolized rutin derivative and L-lysine or an acceptable salt or ester of L-lysine is used to alleviate or treat disorders linked to inflammation and/or auto-immune and/or cutaneous conditions.

16. Use according to any one of the previous claims, **characterized in that** an effective quantity of rutin or glycolized rutin derivative and L-lysine or an acceptable salt or ester of L-lysine is used to alleviate or treat disorders linked to NO, IL-1, TNF- or MCP-1.

17. Use according to claim 1, **characterized in that** a pharmaceutical kit is used, in particular in the form of a blister pack, comprising a first support or receiving compartment or compressed tablet or capsule containing rutin or a glycolized rutin derivative and a second support or receiving compartment or compressed tablet or capsule containing L-lysine or a salt or ester thereof.

## Patentansprüche

1. Verwendung von L-Lysin oder einem akzeptablen Salz oder Ester von L-Lysin zur Herstellung einer Zusammensetzung für eine entzündungshemmende Behandlung umfassend mindestens Rutin oder ein glycolysiertes Rutinderivat, um die entzündungshemmende Aktivität des Rutins oder des glycolysierten Rutinderivats zu steigern.

2. Verwendung nach Anspruch 1, **dadurch gekennzeichnet, dass** für die Herstellung der Zusammensetzung mindestens eine wirksame Menge an Rutin oder glycolysiertem Rutinderivat und von L-Lysin oder einem akzeptablen Salz oder Ester von L-Lysin als Wirkstoff für die Behandlung von Störungen in Zusammenhang mit Entzündungen verwendet wird.

3. Verwendung nach Anspruch 1 oder 2, **dadurch gekennzeichnet, dass** für die Zusammensetzung das Gewichtsverhältnis von Rutin oder glycolysierten Rutinderivatn / L-Lysin oder einem akzeptablen Salz oder Ester von L-Lysin dargestellt in seiner Säureform, zwischen 1:1000 und 100:1, mit Vorteil zwischen 1:500 und 50:1, bevorzugt zwischen 1:250 und 1:1 liegt.

4. Verwendung nach einem der vorhergehenden Ansprüche, **dadurch gekennzeichnet, dass** eine wirksame Menge eines glycolysierten Rutinderivats verwendet wird.

5. Verwendung nach einem der vorhergehenden Ansprüche, **dadurch gekennzeichnet, dass** das Rutin oder das glycolysierte Rutinderivat in gelöster Form in einem Exzipienten, mit Vorteil halbfest oder pastös vorliegt.

6. Verwendung nach einem der vorhergehenden Ansprüche, **dadurch gekennzeichnet, dass** die Zusammensetzung in einer injizierbaren Form, einer Form zur topischen Anwendung, einer Form zur oralen Anwendung oder einer Form zur parenteralen Anwendung vorliegt.

7. Verwendung nach einem der vorhergehenden Ansprüche, **dadurch gekennzeichnet, dass** die Zusammensetzung außer dem L-Lysin mindestens eine weitere Aminosäure umfasst, wobei diese weitere Aminosäure in Form ihrer Säure oder in Form eines Salzes oder Esters vorliegt.

8. Verwendung nach einem der vorhergehenden Ansprüche, **dadurch gekennzeichnet, dass** die Zusammensetzung mindestens ein Antioxidationsmittel umfasst.

9. Verwendung nach einem der vorhergehenden Ansprüche, **dadurch gekennzeichnet, dass** die Zusammensetzung mindestens einen Kristallisationshemmer umfasst.

10. Verwendung nach einem der vorhergehenden Ansprüche, **dadurch gekennzeichnet, dass** die Zusammensetzung in Form einer kontrolliert freisetzbaren Form vorliegt.

11. Verwendung nach einem der vorhergehenden Ansprüche, **dadurch gekennzeichnet, dass** die Zusammensetzung in Form einer Matrix vorliegt, in der das Rutin, das eine oder die mehreren glycolysierten Rutinderivate und das L-Lysin oder ein akzeptables Salz oder Ester des L-Lysins in einer festen, halbfesten oder flüssigen Form dispergiert sind.

12. Verwendung nach einem der vorhergehenden Ansprüche, **dadurch gekennzeichnet, dass** die Zusammensetzung mindestens eine Verbindung ausgewählt aus Polyoxyethylenglycolen, Polyoxypropylenglycolen, Polyoxy(ethylen/propylen)glycolen, Ethern davon und ihren Mischungen umfasst.

13. Verwendung nach einem der vorhergehenden Ansprüche, **dadurch gekennzeichnet, dass** die Zusammensetzung als glycolysiertes Rutinderivat ein Derivat von Rutin in glycolysierter Form extrahiert aus Sophora japonica oder einer Kultur von Zellen von Sophora japonica umfasst.

14. Verwendung nach einem der vorhergehenden Ansprüche, **dadurch gekennzeichnet, dass** die Zusammensetzung einen Extrakt von Sophora japonica und/oder einen glycolysierten Extrakt von 3-Rutinosid von 3,3',4',5,7-Pentahydroxyflavon umfasst, der eine HPLC-Kurve zeigt, die eine Retentionszeit von 27,546 Minuten mit einer maximalen Absorption für Wellenlängen von 255,6 nm und 355,2 nm aufweist.

15. Verwendung nach einem der vorhergehenden Ansprüche, **dadurch gekennzeichnet, dass** eine wirksame Menge von Rutin oder glycolysiertem Rutinderivat und von L-Lysin oder einem akzeptablen Salz oder Ester von L-Lysin zur Linderung oder Behandlung einer oder mehrerer Störungen verwendet wird, die mit einer Entzündung und/oder Autoimmunerkrankungen und/oder Hauterkrankungen in Zusammenhang stehen.

16. Verwendung nach einem der vorhergehenden Ansprüche, **dadurch gekennzeichnet, dass** eine wirksame Menge von Rutin oder glycolysiertem Rutinderivat und von L-Lysin oder einem akzeptablen Salz oder Ester von L-Lysin zur Linderung oder Behandlung von Störungen verwendet wird, die mit NO, IL-1, TNF- oder MCP-1 in Zusammenhang stehen.

17. Verwendung nach Anspruch 1, **dadurch gekennzeichnet, dass** ein pharmazeutisches Kit, insbesondere in Blister-Form, verwendet wird, umfassend einen ersten Träger oder eine Aufnahmevorrichtung oder eine Tablette oder eine Kapsel, die das Rutin oder ein glycolysiertes Rutinderivat enthält, und einen zweiten Träger oder eine Aufnahmevorrichtung oder eine Tablette oder eine Kapsel, die das L-Lysin oder ein Salz oder einen Ester davon enthält.
